Europäisches Patentamt

European Patent Office

Office européen des brevets

(11) Publication number: **0 137 081**
**B1**

(12) # EUROPEAN PATENT SPECIFICATION

(45) Date of publication of patent specification: **08.07.87**

(21) Application number: **83306074.2**

(22) Date of filing: **07.10.83**

(51) Int. Cl.⁴: **B 01 J 8/12,** B 01 J 8/02 // C10G35/12, C10G35/04

(54) Catalytic reactor system and hydrocarbon conversion process utilizing it.

(43) Date of publication of application:
**17.04.85 Bulletin 85/16**

(45) Publication of the grant of the patent:
**08.07.87 Bulletin 87/28**

(84) Designated Contracting States:
**AT BE CH DE FR GB IT LI NL SE**

(56) References cited:
**US-A-2 521 538**
**US-A-2 617 718**
**US-A-3 725 248**
**US-A-3 882 015**
**US-A-4 040 794**
**US-A-4 167 474**

(73) Proprietor: **UOP Inc.**
**10 UOP Plaza Algonquin & Mt. Prospect Roads**
**Des Plaines Illinois 60016 (US)**

(72) Inventor: **Kroushl, Joseph Alfred**
**1643 Bristol Avenue**
**Westchester Illinois 60153 (US)**
Inventor: **Greenwood, Arthur Raymond**
**8201 Elmore Street**
**Niles Illinois 60648 (US)**

(74) Representative: **Hale, Stephen Geoffrey et al**
**J.Y. & G.W. Johnson Furnival House 14/18 High Holborn**
**London WC1V 6DE (GB)**

Courier Press, Leamington Spa, England.

## Description

Background of the invention

The present invention relates to a reactor system and process which is particularly useful in the vapor phase conversion of various hydrocarbon feedstocks. The reactor system provides radial flow contact of a reactant stream with catalyst particles contained in a fixed annular-form bed, or movable as an annular-form bed through said reactor system via gravity flow. The reactor system is adapted to a single stage hydrocarbon conversion process wherein a reactant stream is processed through a single reaction chamber in contact with catalyst particles contained therein, or to a multiple stage hydrocarbon conversion process wherein a reactant stream is processed serially through two or more reaction chambers in contact with catalyst particles which are preferably movable serially through said reaction chamber via gravity flow, said reaction chamber being arranged in stacked or side-by-side configuration or a combination thereof.

Various vapor phase conversion processes have heretofore been effected utilizing a reactor system wherein a reactant stream is processed in radial flow through a vertically positioned annular-form catalyst bed—an arrangement that offers many design and operating advantages, particularly with respect to those vapor phase processes for the conversion of hydrocarbons. Illustrative of a reactor system wherein a reactant stream is caused to flow laterally and radially through an annular-form catalyst bed is that described in U.S. Patent 2,683,654. The reactor system illustrated is intended for a fixed bed operation. A reactant stream charged to a reaction chamber flows from an outer annular-form space created between the chamber walls and the annular-form catalyst bed, said stream flowing laterally and radially through said catalyst bed and into a perforated centerpipe to be discharged from the reaction chamber. U.S. Patent 3,692,496 describes a somewhat related reactor system in that a reactant stream charged to a reaction chamber is caused to flow laterally and radially from an outer annular-form space through an annular-form catalyst section and into an inner or center manifold to be discharged from said chamber. In the latter case, the reactor system comprises stacked reaction chambers (and consequently stacked annular-form catalyst sections) designed to process catalyst particles downwardly via gravity flow from one annular-form catalyst section through the next lower annular-form catalyst section, the catalyst particles being recovered from the lowermost reaction chamber for regeneration. A variation of the last described reactor system appears in U.S. Patent 3,725,248 wherein the annular-form catalyst sections are individually contained in side-by-side reaction chambers, and in U.S. Patent 3,882,015 wherein the reactant stream is reversed to flow laterally and radially from a center reactant conduit through an annular-form catalyst section and into an outer annular-form space formed by the annular-form catalyst section and the reaction chamber walls.

In addition to the foregoing reactor systems in which the reactant stream is processed laterally and radially across an annular-form catalyst bed, reactor systems comprising multiple catalyst beds in a single reactor vessel have been utilized. For example, U.S. Patent 4,040,794 discloses a reactor wherein the reactants flow laterally and radially across an annular-form moving catalyst bed. However, this reactor system also employs baffles attached along the sides of the annular-form catalyst bed to channel reactant flow in such a manner as to create several distinct serially connected catalyst beds from a single bed of catalyst within one reactor vessel. U.S. Patent 2,617,718 discloses a further reactor system of the stationary type comprising a reactor housing with a plurality of catalyst containers located therein. The catalyst containers are depicted as annular-form catalyst beds emplaced within foraminous containers. The inlet and outlet baffles as well as the emplacement of the catalyst containers act to channel reactant flow in such a manner as to create a plurality of catalyst beds in parallel arrangement. In addition, there are baffles situated around and within each catalyst container to promote uniform flow of reactants across the annular-form catalyst beds.

The foregoing reactor systems have heretofore been described with respect to vapor phase conversion processes wherein they are employed to effect a number of catalyst-promoted conversions. Prominent among such conversion processes are the hydrocarbon conversion processes and include catalytic reforming, hydrogenation, hydrocracking, hydrorefining, isomerization, and dehydrogenation, as well as alkylation, transalkylation, steam reforming, and the like. The reactor system of the present invention can be similarly employed, but is of particular advantage with respect to a relatively low pressure operation, such as propane and/or butane dehydrogenation at near-atmospheric pressures.

Brief summary of the invention

The reactor system of the present invention provides for the containment of catalyst particles within a plurality of annular-form catalyst sections contained in a reaction chamber whereby a reactant stream is distributed amongst said plurality of catalyst sections to effect a minimal pressure drop—a feature which is of particular advantage with respect to a relatively low pressure hydrocarbon conversion process. Briefly, the reactor system of this invention comprises two or more vertically positioned annular-form catalyst-retaining sections existing side-by-side in a reaction chamber, each of said catalyst-retaining sections being defined by an inner tubular-form catalyst-retaining screen (typically supported by a perforated or slotted centerpipe) coaxially disposed within a vertically positioned outer tubular-form catalyst-retaining screen. Catalyst particles are preferably movable through the resulting annular-form catalyst sections in a dense phase via gravity flow. The reactant stream may be introduced into the reaction chamber and distributed into each of said annular-form catalyst-retaining sections by way of a fluid flow conduit created by the inner catalyst-retaining screen, the reactant stream then being in each case directed outwardly from said conduit, through the annular-form catalyst-retaining section in a substantially radial flow, and into the reaction chamber void space to be recovered in admixture with the effluent from the other annular-form catalyst-retaining sections contained therein. Alternatively, the reactant stream may be introduced into the reaction chamber void space and distributed into each of said annular-form catalyst-retaining sections across the outer catalyst-retaining screen, the reactant stream being in each case directed inwardly from said outer catalyst screen, through the annular-form catalyst-retaining section in a substantially radial flow, and into the fluid flow conduit defined by the inner catalyst-retaining screen to be recovered in admixture with the effluent from the other reaction chamber annular-form catalyst-retaining sections by way of a manifold. Where the reactor system comprises two or more vertically spaced or stacked reaction chambers, the reactant stream is processed serially through said reaction chamber. Additionally, the reaction chamber of the present invention has associated therewith first and second ports both located in one of said extremities. The first and second ports serve as means for introducing reactants to the reaction chamber and as means for withdrawing the reaction effluents. It should be noted that, in contrast to the reactor system of the present invention, the reactor systems previously described do not employ a plurality of catalyst-retaining sections in a single reaction chamber in conjunction with the advantageous location of the reactant and reaction effluent ports.

In a broad aspect, the present invention provides a reactor system which comprises, in combination, a vertically elongated confined reaction chamber having upper and lower extremities; a first port and a second port associated with the chamber, both located in one of said extremities; said chamber containing a plurality of side-by-side annular-form vertically aligned catalyst-retaining sections positioned therein; each of said catalyst-retaining sections being defined by an inner tubular-form catalyst-retaining screen coaxially disposed within an outer vertically positioned tubular-form catalyst-retaining screen, said inner catalyst-retaining screen further defining a fluid flow conduit within the inner space thereof; said chamber containing a first flow manifold connecting said first port to one extremity of each of said fluid flow conduits each of said catalyst-retaining sections being further defined by two transverse partitions, one connected to the upper extremity of said fluid flow conduit and to the upper perimeter of said outer catalyst-retaining screen, and the other connected to the lower extremity of said fluid flow conduit and to the lower perimeter of said outer catalyst-retaining screen; and, said chamber having a void space in open communication with said second port, and defined by the inner walls of the chamber and the outer surface of the plurality of catalyst-retaining sections contained therein, said void space serving as a second flow manifold connecting the outer surface of the plurality of catalyst-retaining sections to the second port.

Preferably, said reaction chamber further comprises catalyst supply conduits extending downwardly through the upper extremity thereof and in open communication with the upper extremity of each of said annular-form catalyst-retaining sections, and catalyst recovery conduits in open communication with the lower extremity of each of said annular-form catalyst-retaining sections and extending downwardly through the lower extremity of said reaction chamber.

In a particularly preferred embodiment the present invention provides a multiple stage reactor system which comprises, in combination, a vertically elongated confined reactor vessel; at least two vertically spaced reaction chambers within said vessel, each having upper and lower extremities; a first port and a second port associated with each chamber, both located in one of said extremities; each of said chambers containing a plurality of side-by-side annular-form vertically aligned catalyst-retaining sections positioned therein; each of said catalyst-retaining sections being defined by an inner tubular-form catalyst-retaining screen coaxially disposed within an outer vertically positioned tubular-form catalyst-retaining screen, said inner catalyst-retaining screen further defining a fluid flow conduit within the inner space thereof; each of said chambers containing a first flow manifold connecting said first port to one extremity of each of said fluid flow conduits; each of said catalyst-retaining sections being further defined by two transverse partitions, one connected to the upper extremity of said fluid flow conduit and to the upper perimeter of said outer catalyst retaining screen, and the other connected to the lower extremity of said fluid flow conduit and to the lower perimeter of said outer. catalyst-retaining screen; each of said chambers having a void space in open communication with said second port, and defined by the inner walls of said chamber and the outer surface of the plurality of catalyst-retaining sections contained therein, said void space serving as a second flow manifold connecting the outer surface of said plurality of catalyst-retaining sections to said second port; catalyst inlet conduits extending through the upper extremity of the uppermost chamber and connected in open communication with the upper extremity of each of the catalyst-retaining sections contained therein; inter-chamber catalyst conduits connecting the lower extremity of each catalyst-retaining section in each reaction chamber, except the catalyst-retaining sections in the lowermost chamber, in open communication with the upper extremity of a catalyst-retaining section in the next lower chamber; and catalyst withdrawal conduits connected in open communication with the lower extremity of each of the catalyst-retaining sections contained in the lowermost chamber and extending through the lower extremity thereof, whereby catalyst particles are gravitated through said vertically spaced reaction chambers via the plurality of catalyst-retaining sections contained therein.

Preferably, the vertically elongated confined reactor vessel contains three vertically spaced reaction chambers within it. Preferably, the or each reaction chamber of the system contains six side-by-side vertically aligned annular-form catalyst-retaining sections circumferentially spaced therein. Preferably, the first and second ports associated with the or each reaction chamber are in its lower extremity and the first flow manifold connects the first port to the lower extremity of each of the fluid flow conduits contained therein.

The invention also includes a process for the conversion of a hydrocarbon charge stock characterized in that the hydrocarbon charge stock is passed in vapor phase to a reactor system according to the invention. For example, the hydrocarbon feedstock may be subjected to catalytic dehydrogenation in the reactor system in the presence of a dehydrogenation catalyst emplaced within the catalyst-retaining sections.

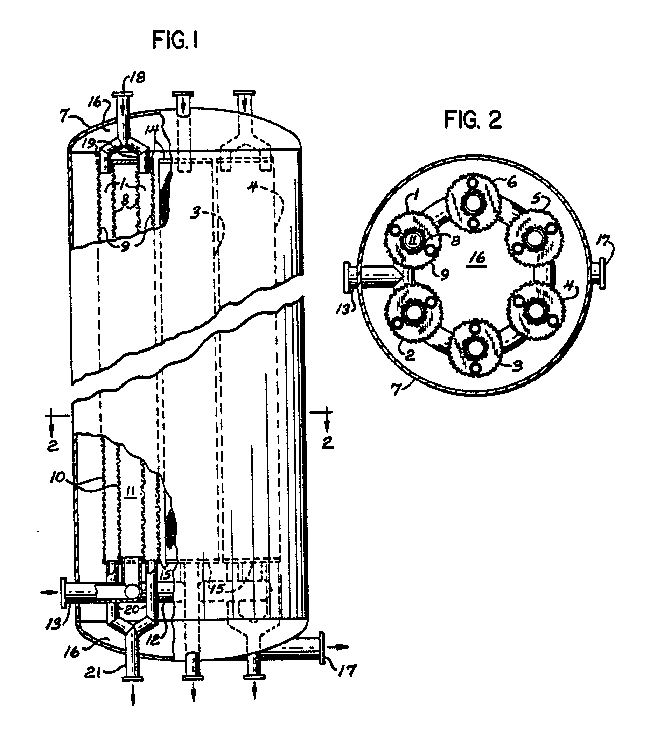

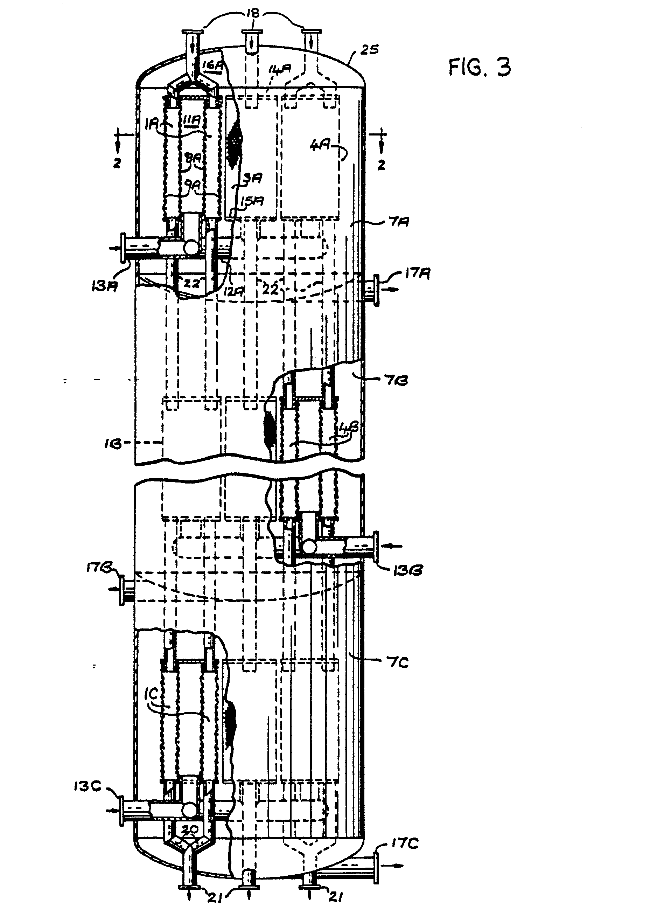

The further description of the reactor system and process of the present invention is presented with reference to the attached schematic drawings. Figures 1 and 3 of the drawings represent side views of two embodiments of reactor systems in accordance with the present invention, partially broken away and sectioned. Figure 2 is a sectional view of the reactor system looking down along the line 2—2 in Figures 1 and 3.

The drawings are presented in illustration of preferred embodiments of the invention and are not intended as a limitation on the scope of the invention as set out in the appended claims. While the drawings depict a first flow manifold connecting first port to the lower extremity of each of a plurality of fluid flow conduits, as well as a second port at the lower extremity of a reaction chamber, it is to be understood that said flow manifold may be connected to the upper extremity of each of said flow conduits and to the first port, with said first and second ports being then located in the upper extremity of the reaction chamber.

Additionally, even though reactant flow across each catalyst-retaining section is depicted as being from the fluid flow conduit radially outward toward the outer catalyst-retaining screen, the flow pattern through the reactor system may be advantageously reversed depending on the specific application. Hence reactant flow across the catalyst-retaining sections may be radially inward from the outer catalyst-retaining screen toward the fluid flow conduit. Also, while a reactant stream is depicted in Figure 1 as being processed through a reactor system comprising a single reaction chamber, it is understood that the reactor system may comprise two or more vertically stacked reaction chambers as depicted in Figure 3, or the reactor system may comprise two or more reaction chambers arranged side-by-side, provision being made in either case for intermediate heating of a reactant stream between said chambers to maintain a desired reaction temperature in the various chambers. In the multiple stage or multiple reaction chamber system, fresh and/or regenerated catalyst particles may be added continuously or intermittently to an initial or top reaction chamber and processed serially or sequentially through the reactor system, said particles being withdrawn from the final or bottom reaction chamber, as the case may be, for subsequent regeneration. Also, the reactor system of this invention may comprise two or more side-by-side reaction chambers whereby fresh and/or regenerated catalyst particles may be added continuously or intermittently to each of two or more reaction chambers, the catalyst particles being withdrawn from each of said reaction chambers to be regenerated separately or in combination. Alternatively, the catalyst particles of a selected reaction chamber may be regenerated periodically while maintaining all reaction chambers, including the selected reaction chamber, onstream at hydrocarbon conversion conditions. In the reactor system comprising stacked reaction chambers, the reactor system will in effect comprise a plurality of annular-form catalyst beds, each of which is movable downwardly from the top reaction chamber and through the reactor system as substantially unbroken or continuous annular-form columns to be recovered from the bottom reaction chamber. Thus, all reaction chambers are maintained onstream while fresh and/or regenerated catalyst particles are added to the top reaction chamber, and used catalyst particles are recovered from the bottom reaction chamber to effect a substantially continuous process.

Referring then to Figures 1 and 2, there are shown six annular-form vertically aligned catalyst-retaining sections numbered 1, 2, 3, 4, 5 and 6, circumferentially spaced within a reaction chamber 7, all of said catalyst-retaining sections being visible in Figure 2. Catalyst-retaining sections 1, 3 and 4 are visible in Figure 1 with catalyst-retaining section 2 and those sections in vertical alignment therewith being not shown to provide a more complete view of first flow manifold 12. The six catalyst-retaining sections are preferably circumferentially spaced as this arrangement permits ready access to each for repair or replacement should the occasion arise. Otherwise, from four to seven catalyst-retaining sections are preferred per reaction chamber. All of said catalyst-retaining sections being substantially identical, the description of catalyst-retaining section 1 will serve to also describe the remaining sections. With reference then to catalyst-retaining section 1, said section comprises an inner tubular-form catalyst-retaining screen 8 coaxially disposed within a vertically positioned outer tubular-form catalyst-retaining screen 9. Both the inner and outer catalyst-retaining screen members comprise perforations 10. The inner tubular-form catalyst-retaining screen 8 further defines a fluid flow conduit 11 within the inner space thereof. A first flow manifold 12 is connected to the lower extremity of each of the fluid flow conduits 11 whereby a reactant stream introduced into the reaction chamber 7 by way of first port 13 is distributed into each of said catalyst-retaining sections. Transverse partition 14 is connected to the upper extremity of fluid flow conduit 11 and to the upper perimeter of outer catalyst-retaining screen 9 while transverse partition 15 is connected to the lower extremity of fluid flow conduit 11 and the lower perimeter of outer catalyst-retaining screen 9. A reactant stream rising through fluid flow conduit 11 is thereby directed laterally and radially outward through annular-form catalyst-retaining section 1 and into void space 16 where the effluent from the plurality of catalyst-retaining sections is collected for discharge through second port 17 in open communication with void space 16. The void space 16 is defined by the interior walls of the reaction chamber 7 and the outer surface of the plurality of the catalyst-retaining screens contained therein. Of course as noted earlier, the flow pattern through the reactor system alternatively may be such that the reactant stream enters via second port 17 into void space 16 thereby flowing laterally and radially inward through annular-form catalyst-retaining section 1 and into fluid flow conduit 11 for eventual withdrawal via first flow manifold 12 and first port 13. Thus first port 13 may be an inlet or outlet port and second port 17 will correspondingly act as an outlet or inlet port. Fresh and/or regenerated catalyst particles are introduced through the upper extremity of the reaction chamber 7 by way of an inlet port 18. The catalyst particles gravitate downwardly through the catalyst conduit 19 which is in open communication with the upper extremity of the annular-form catalyst-retaining section 1. A plurality of conduit outlets into said annular-form catalyst-retaining section are circumferentially spaced to effect a substantially uniform distribution of catalyst particles thereto. The gravitating catalyst particles are withdrawn from the lower extremity of said annular-form catalyst-retaining section by means of catalyst recovery conduit 20. Transverse partition 15 directs catalyst particles, descending through the catalyst-retaining section, into said catalyst recovery conduit. The catalyst particles are recovered from reaction chamber 7 by way of outlet port 21, preferably for regeneration in an on-stream catalyst regeneration facility wherein the catalyst particles are treated as a gravitating moving bed in a continuous type of operation.

Referring now to Figure 3, there is shown a vertically elongated confined reactor vessel 25 having three vertically spaced confined reaction chambers 7A, 7B and 7C. Each of said reaction chambers is further shown as containing six annular-form catalyst-retaining sections in vertical alignment with those of the vertically adjacent reaction chamber or chambers. All of said catalyst-retaining sections are substantially identical to one another and to those shown in Fig. 1, like reference numerals being used to identify like items with the addition of the suffix A, B or C to distinguish items in chambers 7A, 7B and 7C as the case may be. Further description is therefore deemed unnecessary.

Catalyst supply conduits 19, catalyst transfer conduits 22 and catalyst recovery conduits 21 provide for the gravity flow of catalyst particles through the vertically stacked reaction chambers 7 via the plurality of catalyst-retaining sections 1—6 contained therein. As noted above, each of said catalyst-retaining sections contain a lower transverse partition 15 extending between the lower extremity of the fluid flow conduit 11 and the lower perimeter of outer catalyst-retaining screen 9 whereby catalyst particles descending through said catalyst-retaining sections are directed through said catalyst conduits. Using catalyst-retaining section 1A and catalyst-retaining section 1B and 1C in vertical alignment therewith as an example, fresh and/or regenerated catalyst particles are introduced through the uppermost extremity of the uppermost reaction chamber 7A by way of inlet ports 18 and catalyst supply conduits 20. A catalyst supply conduit 20 is shown as connected in open communication with catalyst-retaining section 1A by way of two branches. However, said branches may be but two of several branches circumferentially spaced about the annular-form catalyst-retaining section 1A to ensure a uniform distribution of catalyst particles therein. Inter-chamber catalyst transfer conduit 22 is similarly spaced to provide a substantially uniform gravitational flow of catalyst particles from the uppermost catalyst-retaining section 1A to the next lower catalyst-retaining section 1B in vertical alignment therewith, and from the last mentioned catalyst-retaining section to the vertically aligned lowermost catalyst-retaining section 1C. Catalyst particles are withdrawn from the annular-form catalyst-retaining section 1C by way of catalyst recovery conduit 20 and outlet port 21. The conduits are shown as connected in open communication with the catalyst-retaining section 1C by way of two branches. These again may be but two of several branches, generally from about four to about eight, circumferentially spaced about the lower extremity of the lowermost catalyst-retaining section 1C. The gravity flow of catalyst particles through the vertically stacked reaction chambers via the remaining vertically aligned annular-form catalyst-retaining sections is effected substantially as described with respect to the vertically aligned annular-form catalyst-retaining sections 1A, 1B and 1C.

In the embodiments depicted in the drawings, first port 13 and second port 17 are shown to be situated in the lower extremity of reaction chamber 7 and first flow manifold 12 correspondingly connects first port 13 to the lower extremity of fluid flow conduit 11. Alternatively, both ports may be situated in the upper extremity of the reaction chamber. When both ports are so located, first flow manifold 12 will then correspondingly connect first port 13 to the upper extremity of the fluid flow conduits. By connecting first flow manifold 12 to the extremity of the fluid flow conduits which correspond to the reaction chamber extremity in which first port 13 and second port 17 are located, two major advantages result. First, the amount of internal conduit comprising ths first flow manifold is minimized thereby resulting in reduced cost and lower pressure drop through the reaction chamber. Second, the velocity heads of the vapor in the fluid flow conduits and in the void space surrounding the catalyst-retaining sections become balanced thereby promoting a more uniform flow of vapor across the length of the catalyst-retaining sections. The vapor velocity heads become balanced as a result of the vapor flow path through the fluid flow conduits and through the reaction chamber void space. The velocity head of the vapor decreases as the vapor passes along the length of the fluid flow conduit. Accordingly then, the vapor velocity head is at a maximum at the inlet to the fluid flow conduit and is essentially zero at the terminal end (the upper end in Figures 1 and 3) thereof. As the velocity head of the vapor decreases, the pressure of the vapor increases. Therefore, the vapor pressure at the fluid flow conduit inlet is less than the vapor pressure at the terminal end of the fluid flow conduit and there accordingly exists a vapor pressure gradient along the fluid flow conduit from the inlet to the terminal end thereof. In order to assure uniform flow of vapor across the catalyst beds contained in the annular catalyst-retaining sections, a corresponding vapor pressure gradient must be induced in the void space and therefore, the vapor velocity head in the void space must correspondingly decrease in the same direction as that in the fluid flow conduit to assure a corresponding vapor pressure gradient on the void space side of the catalyst-retaining sections. The vapor flow in the void space must be such that a minimum vapor velocity head therein occurs at a point corresponding to the terminal end of fluid flow conduits and a maximum vapor velocity head therein occurs at a point corresponding to the inlet of the fluid flow conduits. Such a vapor flow will occur in the void space if the flow of vapor therein is in the opposite direction to the flow of vapor in the fluid flow conduit. If, contrary to the above, the vapor flow in the void space is in the same direction as that in the fluid flow conduit, the vapor pressure gradient in the void space will be the opposite of that in the fluid flow conduit and will result in non-uniform flow of vapor across the annular catalyst bed. Accordingly then, the advantages to be derived by situating the first port and the second port in the same extremity of the reaction chamber become apparent. By so situating the ports, it is possible to induce vapor flow patterns through the void space and the fluid flow conduits which result in a more uniform flow of vapor across the annular catalyst bed. Moreover, the improvement in the uniformity of vapor flow will be achieved regardless of whether the first port and second port are both located in the upper or lower extremity of the reaction chamber or whether the flow is outward from the fluid flow conduits to the void space or inward from the void space to the fluid flow conduits.

The reactor system of the present invention is of particular advantage with respect to the conversion of hydrocarbons and in particular the dehydrogenation of hydrocarbons in the presence of a dehydrogenation catalyst—an established and well known hydrocarbon conversion process in the petroleum refining industry. The invention offers special advantage when the hydrocarbon charge stock to be dehydrogenated comprises $C_2+$ normally gaseous hydrocarbons with the desired product comprising the corresponding monoolefins. The monoolefinic products are generally useful as intermediates in the production of other more valuable products, and the catalytic dehydrogenation process is typically utilized in conjunction with various other hydrocarbon conversion processes to yield a desired final product. For example, utilizing liquid petroleum gas (LPG)—a compressed or liquefied gas consisting of propane and butane or mixed butanes—as a starting material, catalytic dehydrogenation can be utilized to produce propylene and/or butylenes in conjunction with an HF alkylation unit wherein said olefins are alkylated with isobutane to produce a high octane motor fuel; or in conjunction with a catalytic condensation unit wherein said olefins are condensed to form tetramers or polymer gasoline; or in conjunction with an etherification unit wherein isobutylene is reacted with methanol to produce methyl t-butyl ether, a highly desirable gasoline additive.

The catalytic dehydrogenation process will preferably utilize a catalytic composite comprising a platinum group metal component, a tin component, and an alkali metal component composited with a porous, high surface area, adsorbent support or carrier material. Of the platinum group metals, i.e., platinum, palladium, ruthenium, rhodium, osmium and iridium, platinum is a preferred catalyst component. The platinum component will generally comprise from about 0.01 to about 2.0 wt.% of the catalytic composite, and the tin component will generally comprise from about 0.01 to about 5 wt.% thereof. Of the alkali metals, i.e., cesium, rubidium, potassium, sodium and lithium, lithium and/or potassium are preferred. The alkali metal will generally constitute from about 0.1 to about 3.5 wt.% of the catalytic composite. One preferred catalytic composite comprises from about 0.1 to about 1.0 wt.% platinum, and from about 0.1 to about 1.0 wt.% tin and from about 0.2 to about 3.0 wt.% lithium or potassium composited with a porous adsorbent support or carrier material having a surface area of from about 25 to about 500 m²/g. The preferred carrier materials are the refractory inorganic oxides with best results being obtained with an alumina support or carrier material.

The catalytic dehydrogenation process herein contemplated is a relatively high temperature operation effected at a temperature of from about 900° to about 1300°F (482—704°C), and preferably from about 1000° to about 1250°F (537—677°C). The process is also a relatively low pressure operation effected at a pressure of from about 0 to about 35 psig (0—2,41 bars gauge), preferably from about 10 to about 30 psig (0,69—2,07 bars gauge). The reactor system of this invention is of still further advantage with respect to the relatively high temperature operation in that the flow pattern may be such that the reactants have minimal exposure to thermal conversion conditions prior to contact with the dehydrogenation catalyst to substantially obviate conversion to other than the desired dehydrogenation products. This is accomplished by distributing the inlet reactant stream directly into each of the fluid flow conduits formed by the inner catalyst-retaining screens thereby creating a reactant flow path outward from the fluid flow conduits. In this manner, the reactant stream is exposed to the high inlet temperature for a relatively brief period while it passes through the first flow manifold and the fluid flow conduits. While the residence time of the reactant stream in the relatively high capacity void space outside of the annular-form catalyst beds is substantially longer, thermal conversion is negligible because of the drop in reactant stream temperature resulting from the endothermic nature of the dehydrogenation reaction.

The reactor system of this invention is of particular advantage with respect to the relatively low pressure operation in that it allows a high throughput with minimal pressure drop. This results from the multiple annular-form catalyst-retaining sections employed herein as opposed to the more conventional unitary annular-form catalyst-retaining sections. Generally from four to seven catalyst-retaining sections per reaction chamber in the process of the invention with six being preferred. Each catalyst-retaining section provides an overall annular-form catalyst bed having a larger cross-sectional area allowing for a relatively shallow catalyst bed. The radially flowing reactant stream thus experiences a minimal pressure drop across the bed. In addition, the relatively low pressure drop facilitates the gravitational flow of catalyst particles through the multiple annular-form catalyst-retaining sections.

Notwithstanding that the catalytic dehydrogenation process involves hydrogen-producing reactions, it has been the practice to charge hydrogen to the reaction zone, typically recycle hydrogen, in admixture with the hydrocarbon feedstock—a practice which has been found to promote catalyst activity as well as activity-stability. Dehydrogenation conditions thus further include a hydrogen to hydrocarbon mole ratio of from about 1 to about 10, and more suitably from about 1 to about 4. The hydrocarbon reactant stream is also suitably charged at a rate to provide a liquid hourly space velocity of from about 2 to about 6.

Referring to Figure 3, in the preferred catalytic dehydrogenation process herein contemplated, a hydrogen/hydrocarbon reactant stream is preheated to provide desired temperature conditions in the uppermost reaction chamber 7A. The reactant stream is introduced into said reaction chamber by way of first port 13A and distributed to each of the annular-form catalyst-retaining sections 1A, 2A, 3A, 4A, 5A and 6A by means of first flow manifold 12A and by way of the fluid flow conduit 11A. The reactant stream then passes radially and laterally through each of the annular-form catalyst-retaining sections in contact with the gravitating catalyst bed and is collected in void space 16A. The reactant stream is recovered through second port 17A, reheated in an external heating means, not shown, and recharged to the next lower reaction chamber 7B. The reactant stream is processed through reaction chamber 7B substantially as described with respect to reaction chamber 7A, the reactant stream being withdrawn from chamber 7B, reheated in a heating means, not shown, and recharged to the lowermost reaction chamber 7C wherein it is similarly processed. The reactant stream is recovered from the lowermost reaction chamber 7C for further processing.

**Claims**

1. A reactor system comprising, in combination:

(a) a vertically elongated confined reaction chamber (7) having upper and lower extremities;

(b) a first port (13) and a second port (17) associated with the chamber (7), both located in one of its extremities;

(c) a plurality of side-by-side annular-form vertically aligned catalyst-retaining sections (1—6) positioned in the chamber (7);

(d) each of the catalyst-retaining sections (1—6) being defined by an inner tubular-form catalyst-retaining screen (8) coaxially disposed within an outer vertically positioned tubular-form catalyst-retaining screen (9), the inner catalyst-retaining screen (8) further defining a fluid flow conduit (11) within the inner space thereof;

(e) the chamber (7) containing a first flow manifold (12) connecting the first port (13) to one extremity of each of the fluid flow conduits (11);

(f) each of the catalyst-retaining sections (1—6) being further defined by two transverse partitions (14, 15), one (14) connected to the upper extremity of the fluid flow conduit (11) and to the upper perimeter of the outer catalyst-retaining screen (9), and the other (15) connected to the lower extremity of the fluid flow conduit (11) and to the lower perimeter of the outer catalyst-retaining screen (9); and

(g) the chamber (7) having a void space (16) in open communication with the second port (17), and defined by the inner walls of the chamber (7) and the outer surface of the plurality of catalyst-retaining sections (1—6) contained therein, the void space (16) serving as a second flow manifold connecting the outer surface of the plurality of catalyst-retaining sections (1—6) to the second port (17).

2. A reactor system as claimed in claim 1, characterized in that the reaction chamber (7) is further provided with catalyst supply conduits (19), extending downwardly through the upper extremity thereof and in open communication with an upper extremity of each of the annular-form catalyst-retaining sections (1—6), and with catalyst recovery conduits (20), in open communication with a lower extremity of each of the annular-form catalyst-retaining sections (1—6) and extending downwardly through the lower extremity of the reaction chamber (7).

3. A reactor system as claimed in claim 1, characterized in that a plurality of reaction chambers (7A, 7B, 7C), each having the features defined in (a)—(g) of claim 1, are positioned in vertically spaced apart fashion in a single vertically elongated confined reaction vessel (25).

4. A multiple-stage reactor system which comprises, in combination:

(a) a vertically elongated confined reactor vessel (25);

(b) at least two vertically spaced reaction chambers (7A—7C) within the vessel (25), each having upper and lower extremities;

(c) a first port (13) and a second port (17) associated with each chamber (7), both located in one of its extremities;

(d) each of the chambers (7) containing a plurality of side-by-side annular-form vertically aligned catalyst-retaining sections (1—6) positioned therein;

(e) each of the catalyst-retaining sections (1—6) being defined by an inner tubular-form catalyst-retaining screen (8) coaxially disposed within an outer vertically positioned tubular-form catalyst-retaining screen (9), the inner catalyst-retaining screen (8) further defining a fluid flow conduit (11) within the inner space thereof;

(f) each of the chambers (7) containing a first flow manifold (12) connecting the first port (13) to one extremity of each of the fluid flow conduits (11);

(g) each of the catalyst-retaining sections (1—6) being further defined by two transverse partitions (14, 15), one (14) connected to the upper extremity of the fluid flow conduit (11) and to the upper perimeter of the outer catalyst-retaining screen (9), and the other (15) connected to the lower extremity of the fluid flow conduit (11), and to the lower perimeter of the outer catalyst-retaining screen (9);

(h) each of the chambers (7) having a void space (16) in open communication with the second port (17), and defined by the inner walls of the chamber (7) and the outer surface of the plurality of catalyst-retaining sections (1—6) contained therein, the void space (16) serving as a second flow manifold connecting the outer surface of the plurality of catalyst-retaining sections (1—6) to the second port (17);

(i) catalyst supply conduits (19) extending through the upper extremity of the uppermost chamber (7A) and connected in open communication with the upper extremity of each of the catalyst-retaining sections (1A—6A) contained therein;

(j) inter-chamber catalyst conduits (22) connecting the lower extremity of each catalyst-retaining section (1—6) in each reaction chamber (7), except the lowermost chamber (7C), in open communication with the upper extremity of a catalyst-retaining section (1—6) in the next lower chamber (7); and

(k) catalyst recovery conduits (20) connected in open communication with the lower extremity of each of the catalyst-retaining sections (1C—6C) contained in the lowermost chamber (7C) and extending through the lower extremity thereof, whereby catalyst particles are gravitated through the vertically spaced reaction chambers (7A, 7B, 7C) via the plurality of catalyst-retaining sections (1—6) contained therein.

5. A reactor system as claimed in claim 4, characterized in that three vertically spaced reaction chambers (7A, 7B, 7C) are present.

6. A reactor system as claimed in any of claims 1 to 5, characterized in that the first port (13) and second port (17) are each located in the lower extremity of the reaction chamber (7) and the first flow manifold (12) connects the first port (13) to the lower extremity of each of the fluid flow conduits (1—6).

7. A reactor system as claimed in any of claims 1 to 6, characterized in that the or each reaction chamber (7) has six side-by-side annular-form vertically aligned catalyst-retaining sections (1—6).

8. A reactor system as claimed in any of claims 1 to 7, characterized in that the first port (13) of the or each chamber (7) is a reactant inlet port and the second port (15) of the or each chamber (7) is a reaction effluent outlet port.

9. A reactor system as claimed in any of claims 1 to 7, characterized in that the first port (13) of the or each chamber (7) is a reaction effluent outlet port and the second port (15) of the or each chamber (7) is a reactant inlet port.

10. A process for the conversion of a hydrocarbon charge stock characterized in that the hydrocarbon charge stock is passed in vapor phase to a reactor system as claimed in any of claims 1 to 9.

**Patentansprüche**

1. Reaktorsystem, bestehend aus einer Kombination von:

a) einer senkrechten länglichen geschlossenen Reaktionskammer (7) mit oberem und unterem Ende,

b) einer jeweils an einem der Enden der Kammer (7) angeordneten, dazugehörigen ersten (13) und zweiten (17) Öffnung,

c) mehreren in der Kammer (7) ringförmig nebeneinander angeordneten, senkrecht ausgerichteten Katalysator-Rückhalteabteilen (1—6),

d) wobei die Katalysator-Rückhalteabteile (1—6) jeweils durch ein koaxial in einem äusseren, senkrecht stehenden rohrförmigen Katalysator-Rückhaltegitter (9) angeordnetes inneres rohrförmiges Katalysator-Rückhaltegitter (8) begrenzt sind, welches ferner in seinem Innenraum eine Fluidströmungsleitung (11) begrenzt,

e) die Kammer (7) einen ersten Strömungsverteiler (12) aufweist, der die erste Öffnung (13) mit je einem Ende der Fluidströmungsleitungen (11) verbindet,

f) die Katalysator-Rückhalteabteile (1—6) jeweils ferner durch zwei Quertrennwände (14, 15) begrenzt sind, von denen die eine (14) mit dem Oberende der Fluidströmungsleitung (11) und dem oberen Umfang des äusseren Katalysator-Rüchhaltegitters (9) und die andere (15) mit dem Unterende der Fluidströmungsleitung (11) und dem unteren Umfang des äusseren Katalysator-Rückhaltegitters (9) verbunden ist, und

g) die Kammer (7) einen Leerraum (16) aufweist, der mit der zweiten Öffnung (17) in Verbindung steht und von den Innenwänden Kammer (7) und der Aussenfläche der darin befindlichen mehreren Katalysator-Rückhalteabteile (1—6) begrenzt ist, wobei der Leerraum (16) als zweiter Strömungsverteiler dient, der die Aussenfläche der mehreren Katalysator-Rückhalteabteile (1—6) m der zweiten Öffnung (17) verbindet.

2. Reaktorsystem nach Anspruch 1, dakurch gekennzeichnet, dass die Reaktionskammer (7) ferner mit Katalysatorzufuhrleitungen (19), die sich durch deren Oberende nach unten erstrecken und je mit dem Oberende eines der ringförmigen Katalysator-Rückhalteabteile (1—6) in Verbindung stehen, und mit Katalysataustragsleitungen (20), die je mit dem Unterende eines der ringförmigen Katalysator-Rückhalteabteile (1—6) in Verbindung stehen und sich durch das Unterende der Reaktionskammer (7) nach unten erstrecken, versehen ist.

3. Reaktorsystem nach Anspruch 1, dadurch gekennzeichnet, dass mehrere, je die Merkmale (a)—(g) nach Anspruch 1 aufweisende Reaktionskammern (7A, 7B, 7C) senkrecht beabstandet in einem einzigen senkrechten länglichen geschlossenen Reaktionsbehälter (25) angeordnet sind.

4. Mehrstufiges Reaktorsystem, bestehend aus einer Kombination von:

a) einem senkrechten länglichen geschlossenen Reaktorbehälter (25),

b) mindestens zwei senkrecht beabstandeten Reaktionskammern (7A—7C) jeweils mit oberen und unteren Enden innerhalb des Behälters (25),

c) jeweils an einem der Enden jeder Kammer (7) angeordneten, dazugehörigen ersten (13) und zweiten (17) Öffnungen,

d) wobei die Kammern (7) jeweils mehrere darin ringförmig nebeneinander angeordnete, senkrecht ausgerichtete Katalysator-Rückhalteabteile (1—6) enthalten,

e) wobei die Katalysator-Rückhalteabteile (1—6) jeweils durch ein koaxial in einem äusseren, senkrecht stehenden rohrförmigen Katalysator-Rückhaltegitter (9) angeordnetes inneres rohrförmiges Katalysator-Rückhaltegitter (8) begrenzt sind, welches ferner in seinem Innernraum eine Fluidströmungsleitung (11) begrenzt,

f) die Kammern (7) jeweils einen ersten Strömungsverteiler (12) aufweisen, der die ersten Öffnung (13) mit je einem Ende der Fluidströmungsleitungen (11) verbindet,

g) die Katalysator-Rückhalteabteile (1—6) jeweils ferner durch zwei Quertrennwände (14, 15) begrenzt sind, von denen die eine (14) mit dem Oberende der Fluidstömungsleitung (11) und dem oberen Umfang des äusseren Katalysator-Rückhaltegitters (9) und die andere (15) mit dem Unterende der Fluidströmungsleitung (11) und dem unteren Umfang des äusseren Katalysator-Rückhaltegitters (9) verbunden ist,

h) die Kammern (7) jeweils einen Leerraum (16) aufweisen, der mit derten Öffnung (17) in Verbindung steht und von den Innenwänden der Kammer (7) und der Aussenfläche der darin befindlichen mehrerer Katalysator-Rückhalteabteile (1—6) begrenzt ist, wobei der Leerraum (16) als zweiter Strömungsvertiler dient, der die Aussenfläche der mehreren Katalysator-Rückhalteabteile (1—6) mit der zweiten Öffnung (17) verbindet,

i) Katalysatorzufuhrleitungen (19) sich durch das Oberende der obersten Kammer (7A) erstrekken und je mit dem Oberende eines der darin befindlichen Katalysator-Rückhalteabteile (1A—6A) in Verbindung stehen,

j) Katalysatorleitungen (22) zwischen den Kammern jeweils das Unterende der Katalysator-Rückhalteabteile (1—6) in jeder Reaktionskammer (7) ausser der untersten Kammer (7C) mit dem Oberende eines Katalysator-Rückhalteabteils (1—6) in der nächstniedrigeren Kammer (7) verbinden und

k) Katalysatoraustragsleitungen (20) jeweils mit dem Unterende der in der untersten Kammer (7C) befindlichen Katalysator-Rückhalteabteile (1C—6C) in Verbindung stehen und sich durch deren Unterende erstrecken, wodurch Katalysatorteilchen sich unter Schwerkraft durch die senkrecht beabstandeten Reaktionskammern (7A, 7B, 7C) über dir mehreren, darin enthaltenen Katalysator-Rückhalteabteile (1—6) bewegen.

5. Reaktorsystem nach Anspruch 4, dadurch gekennzeichnet, dass drei senkrecht beabstandete Reaktionskammern (7A, 7B, 7C) vorhanden sind.

6. Reaktorsystem nach einem der Ansprüche 1 bis 5, dadurch gekennzeichnet, dass die erste Öffnung (13) und die zweite Öffnung (17) jeweils im Unterende der Reaktionskammer (7) angeordnet sind und der erste Strömungsverteiler (12) die erste Öffnung (13) mit dem Unterende je einer der Fluidströmungsleitungen (1—6) verbindet.

7. Reaktorsystem nach einem der Ansprüche 1 bis 6, dadurch gekennzeichnet, dass die bzw. jede Reaktionskammer (7) sechs senkrecht ausgerichtete Katalysator-Rückhalteabteile (1—6) ringförmig nebeneinander aufweist.

8. Reaktorsystem nach einem der Ansprüche 1 bis 7, dadurch gekennzeichnet, dass die erste Öffnung (13) der bzw. jeder Kammer (7) eine Einlassöffnung für Reaktanden und die zweite Öffnung (15) der bzw. jeder Kammer (7) eine Auslassöffnung für Reaktionsprodukt ist.

9. Reaktorsystem nach einem der Ansprüche 1 bis 7, dadurch gekennzeichnet, dass die erste Öffnung (13) der bzw. jeder Kammer (7) eine Auslassöffnung für Reaktionsprodukt und die zweite Öffnung (15) der bzw. jeder Kammer (7) eine Einlassöffnung für Reaktanden ist.

10. Verfahren zum Umwandlung eines Kohlenwasserstoffeinsatzmaterials, dadurch gekennzeichnet, dass dieses in Dampfphase durch ein Reaktorsystem nach einem der Ansprüche 1 bis 9 geleitet wird.

**Revendications**

1. Un système de réacteur comportant, en combinaison:

(a) une chambre de réaction enclose, allongée verticalement (7) ayant des extrémités supérieure et inférieure;

(b) un premier orifice (13) et un second orifice (17) associés à la chambre (7), situés tous les deux à l'une de ses extrémités;

(c) plusieurs sections de forme annulaire retenant le catalyseur et alignées verticalement l'une à côté de l'autre (1—6) positionnées dans la chambre (7);

(d) chacune des sections retenant le catalyseur (1—6) étant définie par un écran retenant le catalyseur, de forme tubulaire, interne (8), disposé coaxialement à l'intérieur d'un écran retenant le catalyseur, de forme tubulaire, positionné verticalement, externe (9), l'écran retenant le catalyseur interne (8) définissant en outre une conduite d'écoulement de fluide (11) à l'intérieur de l'espace interne de celui-ci;

(e) la chambr76 contenant un premier collecteur d'écoulement (12) reliant le premier orifice (13) à une extrémité de chacune des conduites d'écoulement de fluide (11);

(f) chacune des sections retenant le catalyseur (1—6) étant définie en outre par deux partitions transversales (14, 15), l'une (14) reliée à l'extrémité supérieure de la conduite d'écoulement de fluide (11) et au périmètre supérieur de l'écran retenant le catalyseur, externe (9) et l'autre (15) reliée à l'extrémité inférieure de la conduite d'écoulement de fluide (11) et au périmètre inférieur de l'écran retenant le catalyseur, externe (9); et

(g) la chambre (7) ayant un espace vide (16) en communication ouverte avec le second orifice (17), et défini par les parois internes de la chambre (7) et les faces externes des plusieurs sections retenant le catalyseur (1—6) contenues dans celle-ci, l'espace vide (16) servant de second collecteur d'écoulement reliant la face externe de la pluralité des sections retenant le catalyseur (1—6) au second orifice (17).

2. Un système de réacteur selon la revendication 1, caractérisé en ce que la chambre de réaction (7) est munie en outre de conduites d'alimentation en catalyseur (19) s'étendant vers le bas à travers l'extrémité supérieure de celle-ci et en communication ouverte avec une extrémité supérieure de chacune des sections de forme annulaire retenant le catalyseur (1—6), et des conduites de récupération de catalyseur (20), en communication ouverte avec une extrémité inférieure de chacune des sections de forme annulaire contenant le catalyseur (1—6) et s'étendant vers le base à travers l'extrémité inférieure de la chambre de réaction (7).

3. Un système de réacteur selon la revendication 1, caractérisé en ce que plusieurs chambres de réaction (7A, 7B, 7C), ayant chacune les caractéristiques définies en (a)—(g) de la revendication 1, sont positionnées de façon espacée verticalement dans un cuve de réaction enclose unique, allongée verticalement (25).

4. Un système de réacteur à plusieurs étapes qui comprend, en combinaison:

(a) une cuve de réaction enclose, allongée verticalement (25);

(b) au moins deux chambres de réaction espacées verticalement (7A—7C) à l'intérieur de la cuve (25), ayant chacune des extrémités supérieure et inférieure;

(c) un premier orifice (13) et un second orifice (17) associés à chaque chambre (7) situés tous les deux à l'une de ses extrémités;

(d) chacune des chambres (7) contenant plusieurs sections de forme annulaire retenant le catalyseur alignées verticalement, l'une à côté de l'autre (1—6), positionnées en son intérieur;

(e) chacune des sections retenant le catalyseur (1—6) étant définie par un écran retenant le catalyseur, de forme tubulaire, interne (8) disposé coaxialement à l'intérieur d'un écran retenant le catalyseur, de forme tubulaire, positionné verticalement, externe (9), l'écran retenant le catalyseur interne (8) définissant en outre une conduite d'écoulement de fluide (11) à l'intérieur de l'espace intene de celle-ci;

(f) chacune des chambres (7) contenant un premier collecteur d'écoulement (12) reliant le premier orifice (13) à une extrémité de chacune des conduite d'écoulement de fluide (11);

(g) chacune des sections retenant le catalyseur (1—6) étant définie en outre par deux partitions transversales (14, 15), l'une (14) reliée à l'extrémité supérieure de la conduite d'écoulement de fluide (11) et au périmètre supérieur de l'écran retenant le catalyseur, externe (9), et l'autre (15) reliée à l'extrémité inférieure de la conduite d'écoulement de fluide (11), et au périmètre inférieur de l'écran retenant le catalyseur, externe (9);

(h) chacune des chambres (7) ayant un espace vide (16) en communication ouverte avec le second orifice (17), et défini par les parois internes de la chambre (7) et la face externe des plusieurs sections retenant le catalyseur (1—6) contenues dans celle-ci, l'espace vide (16) servant de second collecteur d'écoulement reliant la face externe de la pluralité des sections retenant le catalyseur (1—6) au second orifice (17);

(i) des conduits d'alimentation en catalyseur (19) s'étendant à travers l'extrémité supérieure de la chambre la plus haute (7A) et reliées en communication ouverte avec l'extrémité supérieure de chacune des sections retenant le catalyseur (1A—6A) contenues dans celle-ci;

(j) des conduites de catalyseur inter-chambres (22) reliant l'extrémité inférieure de chaque section retenant le catalyseur (1—6) dans chaque chambre de réaction (7), à l'exception de la chambre plus basse (7C), en communication ouverte avec l'extrémité supérieure d'une section retenant le catalyseur (1—6) dans la chambre immédiatement intérieure (7); et

(k) des conduites de récupération de catalyseur (20) reliées en communication ouverte à l'extrémité inférieure de chacune des sections retenant le catalyseur (1C—6C) contenues dans la chambre la plus basse (7C) et s'étendant à travers l'extrémité inférieure de celle-ci, par lesquelles on fait passer des particules de catalyseur par gravité à travers les chambres de réaction espacées verticalement (7A, 7B, 7C) à travers la pluralité des sections retenant le catalyseur (1—6) contenues dans celles-ci.

5. Un système de réacteur selon la revendication 4, caractérisé en ce que trois chambres de réactions espacées verticalement (7A, 7B, 7C) sont présentes.

6. Un système de réacteur selon l'une quelconque des revendications 1 à 5, caractérisé en ce que le premier orifice (13) et le second orifice (17) sont tous deux situés dans l'extrémité inférieure de la chambre de réaction (7) et le premier collecteur d'écoulement (12) relie le premier orifice (13) à l'extrémité inférieure de chacune des conduites d'écoulement de fluide (1—6).

7. Un système de réacteur selon l'une quelconque des revendications 1 à 6, caractérisé en ce que la ou chaque chambre de réaction (7) comporte six sections de forme annulaire retenant le catalyseur alignées verticalement, l'une à côté de l'autre (1—6).

8. Un système de réacteur selon l'une quelconque des revendications 1 à 7, caractérisé en ce que le premier orifice (13) de la ou de chaque chambre (7) est un orifice d'entrée des corps regissants et le second orifice (15) de la ou de chaque chambre (7) est un orifice de sortie de l'effluent de réaction.

9. Un système de réacteur selon l'une quelconque des revendications 1 à 7, caractérisé en ce que le premier orifice (13) de la ou de chaque chambre (7) est un orifice de sortie d'effluent de réaction et le second orifice (15) de la ou de chaque chambre (7) est un orifice d'entrée des corps réagissants.

10. Un procédé de transformation d'une charge d'alimentation hydrocarbonée caractérisé en ce que l'on fait passer la charge d'alimentation hydrocarbonée en phase vapeur dans un système de réacteur selon l'une quelconque des revendications 1 à 9.

## FIG. I

## FIG. 2

FIG. 3